(19) Europäisches Patentamt
European Patent Office
Office européen des brevets

(11) EP 1 748 986 B1

(12) **EUROPEAN PATENT SPECIFICATION**

(45) Date of publication and mention of the grant of the patent:
**01.12.2010 Bulletin 2010/48**

(21) Application number: **05749325.6**

(22) Date of filing: **27.05.2005**

(51) Int Cl.:
***C07D 213/00*** *(2006.01)*

(86) International application number:
**PCT/EP2005/052418**

(87) International publication number:
**WO 2005/118541 (15.12.2005 Gazette 2005/50)**

(54) **HETEROCYCLIC COMPOUNDS AND THEIR USE AS ALDOSTERONE SYNTHASE INHIBITORS**

HETEROCYCLISCHE VERBINDUNGEN UND DEREN VERWENDUNG ALS ALDOSTERONSYNTHASEINHIBITOREN

COMPOSES HETEROCYCLIQUES ET LEUR UTILISATION COMME INHIBITEURS DE L'ALDOSTERONE SYNTHASE

(84) Designated Contracting States:
**AT BE BG CH CY CZ DE DK EE ES FI FR GB GR HU IE IS IT LI LT LU MC NL PL PT RO SE SI SK TR**

(30) Priority: **28.05.2004 CH 9162004**
**09.07.2004 CH 11572004**

(43) Date of publication of application:
**07.02.2007 Bulletin 2007/06**

(73) Proprietor: **Novartis AG**
**4056 Basel (CH)**

(72) Inventors:
- **HEROLD, Peter**
**CH-4055 Basel (CH)**
- **MAH, Robert**
**CH-4132 Muttenz (CH)**
- **TSCHINKE, Vincenzo**
**CH-4102 Binningen (CH)**
- **SCHUMACHER, Christoph**
**CH-4126 Bettingen (CH)**
- **BEHNKE, Dirk**
**79639 Grenzach-Wyhlen (DE)**
- **QUIRMBACH, Michael**
**CH-4054 Basel (CH)**

(74) Representative: **Warner, James Alexander et al**
**Carpmaels & Ransford**
**One Southampton Row**
**London**
**WC1B 5HA (GB)**

(56) References cited:
**EP-A- 0 356 673** **EP-A- 0 366 609**
**EP-A- 0 401 707** **EP-A- 0 426 225**
**WO-A-93/15079**

- **PATENT ABSTRACTS OF JAPAN vol. 1997, no. 07, 31 July 1997 (1997-07-31) -& JP 09 071586 A (YAMANOUCHI PHARMACEUT CO LTD), 18 March 1997 (1997-03-18)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The invention relates to novel heterocycles, to a process for preparing the compounds of the invention, to pharmaceutical products containing them, and to their use as active pharmaceutical ingredients, in particular as aldosterone synthase inhibitors.

JP 09071586 A discloses compounds useful for treating chronic cardiac insufficiency, myocard fibrosis, primary hyperaldosteronism and other diseases.

EP-A-0366609 relates to bicyclic imidazole compounds for the treatment of hyperaldosteronism.

The present invention relates firstly to compounds of the general formula

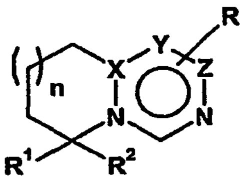

(I)

in which
X is C;
Y is C;
Z is a bond;
R is hydrogen, $C_1$-$C_8$-alkyl, $C_1$-$C_8$-alkoxy-$C_0$-$C_4$-alkyl, halogen or trifluoromethyl;
$R^1$ is unsaturated heterocyclyl-$C_0$-$C_4$-alkyl, which radical is unsubstituted or substituted by 1-4 $C_1$-$C_8$-alkyl, $C_0$-$C_8$-alkylcarbonyl, $C_1$-$C_8$-alkylsulfonyl, halogen, cyano, oxo, tri-$C_1$-$C_4$-alkylsilyl, trifluoromethoxy, trifluoromethyl, $C_0$-$C_8$-alkylcarbonylamino, $C_0$-$C_8$-alkylearbonyl-$C_1$-$C_8$-alkylamino, carbamoyl, mono- or di-$C_1$-$C_8$-alkylaminocarbonyl, carboxy-$C_0$-$C_4$-alkyl, $C_1$-$C_8$-alkoxy, $C_1$-$C_8$-alkoxycarbonyl, heterocyclyl or aryl, where heterocyclyl or aryl is unsubstituted or substituted by 1-4 $C_1$-$C_8$-alkyl, $C_0$-$C_8$-alkylcarbonyl, $C_1$-$C_8$-alkylsulfonyl, halogen, cyano, oxo, tri-$C_1$-$C_4$-alkylsilyl, trifluoromethoxy, trifluoromethyl, $C_0$-$C_8$-alkylcarbonylamino, $C_0$-$C_8$-alkylcarbonyl-$C_1$-$C_8$-alkylamino, carbamoyl, mono- or di-$C_1$-$C_8$-alkylaminocarbonyl, carboxy-$C_0$-$C_4$-alkyl, $C_1$-$C_8$-alkoxy or $C_1$-$C_8$-alkoxycarbonyl;
$R^2$

a) is hydrogen; or
b) is $C_1$-$C_8$-alkyl, $C_3$-$C_8$-cycloalkyl, halogen, carboxy-$C_1$-$C_4$-alkyl, $C_1$-$C_4$-alkoxycarbonyl-$C_1$-$C_4$-alkyl, $C_0$-$C_4$-alkylcarbonyl, aryl-$C_0$-$C_4$-alkyl or unsaturated heterocyclyl-$C_1$-$C_4$-alkyl, which radicals are unsubstituted or substituted by 1-4 $C_1$-$C_8$-alkyl, $C_0$-$C_8$-alkylcarbonyl, $C_1$-$C_8$-alkylsulfonyl, halogen, cyano, oxo, tri-$C_1$-$C_4$-alkylsilyl, trifluoromethoxy, trifluoromethyl, $C_0$-$C_8$-alkylcarbonylamino, $C_0$-$C_8$-alkylcarbonyl-$C_1$-$C_8$-alkylamino, carbamoyl, mono- or di-$C_1$-$C_8$-alkylaminocarbonyl, carboxy-$C_0$-$C_4$-alkyl, $C_1$-$C_8$-alkoxy, $C_1$-$C_8$-alkoxycarbonyl, heterocyclyl or aryl, where heterocyclyl or aryl is unsubstituted or substituted by 1-4 $C_1$-$C_8$-alkyl, $C_0$-$C_8$-alkylcarbonyl, $C_1$-$C_8$-alkylsulfonyl, halogen, cyano, oxo, tri-$C_1$-$C_4$-alkylsilyl, trifluoromethoxy, trifluoromethyl, $C_0$-$C_8$-alkylcarbonylamino, $C_0$-$C_8$-alkylcarbonyl-$C_1$-$C_8$-alkylamino, carbamoyl, mono- or di-$C_1$-$C_8$-alkylaminocarbonyl, carboxy-$C_0$-$C_4$-alkyl, $C_1$-$C_8$-alkoxy or $C_1$-$C_8$-alkoxycarbonyl;

n is a number 0, 1 or 2;
and pharmaceutically usable salts thereof,
where, if $R^2$ is hydrogen, $R^1$ is not benzoimidazolyl or benzotriazolyl.

The term aryl stands for an aromatic hydrocarbon radical which generally comprises 5-14, preferably 6-10, carbon atoms and is, for example, phenyl, indenyl, e.g. 2- or 4-indenyl, or naphthyl, e.g. 1- or 2-naphthyl. Aryl having 6-10 carbon atoms is preferred, especially phenyl or 1- or 2-naphthyl. Said radicals may be unsubstituted or substituted one or more times, e.g. once or twice, it being possible for the substituent to be in any position, e.g. in the o, m or p position of the phenyl radical or in the 3 or 4 position of the 1- or 2-naphthyl radical, and it also being possible for a plurality of identical or different substituents to be present

The term heterocyclyl stands for an unsaturated, 7-12-membered, particularly preferably 9-10-membered, bicyclic ring system, in each case comprising an N, O or S atom in at least one ring, it also being possible for an additional N, O or S atom to be present in one ring. Said radicals may be unsubstituted or substituted one or more times, e.g. once or twice, it also being possible for a plurality of identical or different substituents to be present. $R^1$ as an unsaturated, monocyclic S-containing-heterocycle radical is less preferred.

Unsaturated bicyclic heterocyclyl-$C_0$-$C_4$-alkyl is for example benzofuranyl, benzothiophenyl, indazolyl, indolyl, isoquinolinyl or quinolinyl.

$C_1$-$C_8$-Alkyl may be straight-chain or branched and/or bridged and is, for example, methyl, ethyl, propyl, isopropyl, butyl, isobutyl, secondary butyl, tertiary butyl, or a pentyl, hexyl or heptyl group.

$C_1$-$C_8$-Alkoxy is, for example, $C_1$-$C_5$-alkoxy such as methoxy, ethoxy, propyloxy, isopropyloxy, butyloxy, isobutyloxy, secondary butyloxy, tertiary butyloxy or pentyloxy, but may also be a hexyloxy or heptyloxy group.

$C_1$-$C_8$-Alkoxy-$C_0$-$C_4$-alkyl is, in addition to the meanings mentioned for $C_1$-$C_8$-alkoxy, for example $C_1$-$C_5$-alkoxy-$C_1$-$C_4$-alkyl such as methoxyethyl, ethoxyethyl, propyloxymethyl, isopropyloxybutyl, butyloxymethyl, isobutyloxyethyl, secondary butyloxypropyl, tertiary butyloxybutyl or pentyloxymethyl, but may also be a hexyloxymethyl or heptyloxymethyl group.

$C_1$-$C_8$-Alkoxycarbonyl is preferably $C_1$-$C_5$-alkoxycarbonyl such as methoxycarbonyl, ethoxycarbonyl, propyloxycarbonyl, isopropyloxycarbonyl, butyloxycarbonyl, isobutyloxycarbonyl, secondary butyloxycarbonyl or tertiary butyloxycarbonyl. $C_0$-$C_8$-Alkylcarbonyl is, for example, formyl, acetyl, propionyl, propylcarbonyl, isopropylcarbonyl, butylcarbonyl, isobutylcarbonyl, secondary butylcarbonyl or tertiary butylcarbonyl.

$C_1$-$C_4$-Alkoxycarbonyl-$C_1$-$C_4$-alkyl is, for example, methoxycarbonyl- or ethoxycarbonylmethyl, 2-methoxycarbonyl- or 2-ethoxycarbonylethyl, 3-methoxycarbonyl- or 3-othoxycarbonylpropyl or 4-ethoxycarbonylbutyl.

$C_0$-$C_8$-Alkylcarbonyl is, for example, formyl, acetyl, propionyl, propylcarbonyl, isopropylcarbonyl, butylcarbonyl, isobutylcarbonyl, secondary butylcarbonyl or tertiary butylcarbonyl.

Carboxy-$C_1$-$C_4$-alkyl is, for example, carboxymethyl, 2-carboxyethyl, 2- or 3-carboxypropyl, 2-carboxy-2-methylpropyl, 2-carboxy-2-ethylbutyl or 4-carboxybutyl, in particular carboxymethyl.

$C_3$-$C_8$-Cycloalkyl is, for example, cyclopentyl, cyclohexyl or cycloheptyl, also cyclopropyl, cyclobutyl or cyclooctyl.

Mono- or di-$C_1$-$C_8$-alkylaminocarbonyl is, for example, $C_1$-$C_4$-alkylaminocarbonyl such as methylaminocarbonyl, ethylaminocarbonyl, propylaminocarbonyl or butylaminocarbonyl, or di-$C_1$-$C_4$-alkylaminocarbonyl such as dimethylaminocarbonyl, N-methyl-N-ethylaminocarbonyl, diethylaminocarbonyl, N-methyl-N-propylaminocarbonyl or N-butyl-N-methylaminocarbonyl.

$C_0$-$C_8$-Alkylcarbonylamino is, for example, formylamino, acetylamino, propionylamino, propylcarbonylamino, isopropylcarbonylamino, butylcarbonylamino, isobutylcarbonylamino, secondary butylcarbonylamino or tertiary butylcarbonylamino.

$C_0$-$C_8$-Alkylcarbonyl-$C_1$-$C_8$-alkylamino is, for example, formyl-, acetyl-, propionyl-, propylcarbonyl-, isopropylcarbonyl-, butylcarbonyl-, isobutylcarbonyl-, secondary butylcarbonyl- or tertiary butylcarbonyl-methylamino, formyl-, acetyl-, propionyl-, propylcarbonyl-, isopropylcarbonyl-, butylcarbonyl-, isobutylcarbonyl-, secondary butylcarbonyl- or tertiary butylcarbonyl-ethylamino, formyl-, acetyl-, propionyl-, propylcarbonyl-, isopropylcarbonyl-, butylcarbonyl-, isobutylcarbonyl-, secondary butylcarbonyl- or tertiary butylcarbonyl-propylamino or formyl-, acetyl-, propionyl-, propylcarbonyl-, isopropylcarbonyl-, butylcarbonyl-, isobutylcarbonyl-, secondary butylcarbonyl- or tertiary butylcarbonyl-butylamino.

Halogen is, for example, fluorine, chlorine, bromine or iodine.

The compound groups mentioned below are not to be regarded as closed; on the contrary, parts of these compound groups may be replaced by one another or by the definitions given above, or be omitted, in a meaningful way, e.g. to replace general by more specific definitions.

Preferred compounds of the formula (I) are compounds of the general formulae

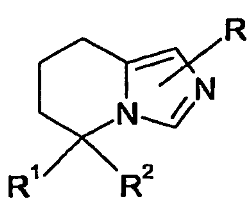

(Ia)

where the meanings of the substituents R, $R^1$ and $R^2$ are as indicated for compounds of the formula (I).

R is preferably hydrogen, $C_1$-$C_8$-alkyl, halogen or trifluoromethyl, particularly preferably hydrogen or methyl.

$R^1$ is preferably indolyl, indazolyl, benzofuranyl, benzothiophenyl or isoquinolinyl, which radicals are unsubstituted or substituted by 1-4 $C_1$-$C_8$-alkyl, $C_0$-$C_8$-alkylcarbonyl, halogen, cyano, oxo, trifluoromethyl, $C_0$-$C_8$-alkylcarbonylamino, $C_0$-$C_8$-alkylcarbonyl-$C_1$-$C_8$-alkylamino, carbamoyl, mono- or di-$C_1$-$C_8$-alkylaminocarbonyl, carboxy-$C_0$-$C_4$-alkyl, $C_1$-$C_8$-alkoxy, $C_1$-$C_8$-alkoxycarbonyl, heterocyclyl or aryl, particularly preferably by cyano, acetyl, oxazolyl, thiazolyl, thiophenyl or pyrrolidinyl.

$R^2$ is preferably hydrogen, halogen, $C_1$-$C_8$-alkyl, aryl-$C_0$-$C_4$-alkyl or unsaturated heterocyclyl-$C_0$-$C_4$-alkyl, particularly preferably hydrogen or $C_1$-$C_3$-alkyl.

Examples of very particularly preferred compounds of the general formulae (I) and (Ia) are therefore those in which

R is hydrogen or methyl;

$R^1$ is indolyl, indazolyl, benzofuranyl, benzothiophenyl or isoquinolinyl, which radicals are unsubstituted or substituted by 1-4 $C_1$-$C_8$-alkyl, $C_0$-$C_8$-alkylcarbonyl, halogen, cyano, oxo, trifluoromethyl, $C_0$-$C_8$-alkylcarbonylamino, $C_0$-$C_8$-alkylcarbonyl-$C_1$-$C_8$-alkylamino, carbamoyl, mono- or di-$C_1$-$C_8$-alkylaminocarbonyl, carboxy-$C_0$-$C_4$-alkyl, $C_1$-$C_8$-alkoxy, $C_1$-$C_8$-alkoxycarbonyl, heterocyclyl or aryl, particularly preferably by cyano, acetyl, oxazolyl, thiazolyl, thiophenyl or pyrrolidinyl; and

$R^2$ is hydrogen or $C_1$-$C_3$-alkyl.

The compounds of the formula (I) which have at least one asymmetric carbon atom can exist in the form of optically pure enantiomers, mixtures of enantiomers or as racemates. Compounds having a second asymmetric carbon atom can exist in the form of optically pure diastereomers, mixtures of diastereomers, diastereomeric racemates, mixtures of diastereomeric racemates or as meso compounds. The invention includes all these forms. Mixtures of enantiomers, racemates, mixtures of diastereomers, diastereomeric racemates or mixtures of diastereomeric racemates can be fractionated by conventional methods, e.g. by racemate resolution, column chromatography, thin-layer chromatography, HPLC and the like.

The term "pharmaceutically usable salts" includes salts with inorganic or organic acids, such as hydrochloric acid, hydrobromic acid, nitric acid, sulphuric acid, phosphoric acid, citric acid, formic acid, maleic acid, acetic acid, succinic acid, tartaric acid, methanesulphonic acid, p-toluenesulphonic acid and the like. Salts of compounds having salt-forming groups are, in particular, acid addition salts, salts with bases or, if a plurality of salt-forming groups is present, optionally also mixed salts or inner salts.

The compounds of the formula (I) can be prepared in a manner analogous to preparation processes disclosed in the literature. Details of the specific preparation variants can be found in the examples.

The compounds of the formula (I) can also be prepared in optically pure form. Separation into antipodes is possible by methods known per se, either preferably at an early stage of the synthesis by salt formation with an optically active acid such as, for example, (+)- or (-)-mandelic acid and separation of the diastereomeric salts by fractional crystallization or preferably at a rather late stage by derivatization with a chiral auxiliary component such as, for example, (+)- or (-)-camphanyl chloride, and separation of the diastereomeric products by chromatography and/or crystallization and subsequent cleavage of the linkage to the chiral auxiliary. The pure diastereomeric salts and derivatives can be analyzed to determine the absolute configuration of the contained compound using conventional spectroscopic methods, a particularly suitable method being single-crystal X-ray spectroscopy.

Salts are primarily the pharmaceutically usable or nontoxic salts of compounds of the formula (I). Such salts are formed for example by compounds of the formula (I) having an acidic group, e.g. a carboxy or sulpho group, and are, for example, salts thereof with suitable bases, such as nontoxic metal salts derived from metals of group Ia, Ib, IIa and IIb of the Periodic Table of Elements, e.g. alkali metal, in particular lithium, sodium or potassium salts, alkaline earth metal salts, for example magnesium or calcium salts, also zinc salts or ammonium salts, and those salts formed with organic amines such as optionally hydroxy-substituted mono-, di- or trialkylamines, in particular mono-, di- or tri-lower-alkylamines, or with quaternary ammonium bases, e.g. methyl-, ethyl-, diethyl- or triethylamine, mono-, bis- or tris(2-hydroxy-lower-alkyl)amines such as ethanol-, diethanol- or triethanolamine, tris(hydroxymethyl)methylamine or 2-hydroxy-tertiary-butylamine, N,N-di-lower alkyl-N-(hydroxy-lower-alkyl)amine, such as N,N-dimethyl-N-(2-hydroxyethyl) amine, or N-methyl-D-glucamine, or quaternary ammonium hydroxides such as tetrabutylammonium hydroxide. The compounds of the formula (I) having a basic group, e.g. an amino group, can form acid addition salts, e.g. with suitable inorganic acids, e.g. hydrohalic acid such as hydrochloric acid, hydrobromic acid, sulphuric acid with replacement of one or both protons, phosphoric acid with replacement of one or more protons, e.g. orthophosphoric acid or metaphosphoric acid, or pyrophosphoric acid with replacement of one or more protons, or with organic carboxylic, sulphonic or phosphonic acids or N-substituted sulphamic acids, e.g. acetic acid, propionic acid, glycolic acid, succinic acid, maleic acid, hydroxymaleic acid, methylmaleic acid, fumaric acid, malic acid, tartaric acid, gluconic acid, glucaric acid, glucuronic acid, citric acid, benzoic acid, cinnamic acid, mandelic acid, salicylic acid, 4-aminosalicylic acid, 2-phenoxybenzoic acid, 2-acetoxybenzoic acid, embonic acid, nicotinic acid, isonicotinic acid, also amino acids such as, for example, $\alpha$-amino acids, and methanesulphonic acid, ethanesulphonic acid, 2-hydroxyethanesulphonic acid, ethane-1,2-disulphonic acid, benzenesulphonic acid, 4-toluenesulphonic acid, naphthalene-2-sulphonic acid, 2- or 3-phosphoglycerate, glucose 6-phosphate, N-cyclohexylsulphamic acid (to form cyclamates) or with other acidic organic compounds such as ascorbic acids. Compounds of the formula (I) having acidic and basic groups can also form inner salts.

Pharmaceutically unsuitable salts can also be used for isolation and purification.

The compounds of the formula (I) also include compounds in which one or more atoms are replaced by their stable, nonradioactive isotopes; for example a hydrogen atom by deuterium.

Prodrug derivatives of the compounds described above are derivatives thereof which on use in vivo release the original compound through a chemical or physiological process. A prodrug may be converted into the original compound for example when a physiological pH is reached or by enzymatic conversion. Examples of possible prodrug derivatives are esters of freely available carboxylic acids, S- and O-acyl derivatives of thiols, alcohols or phenols, where the acyl group is as defined above. Preference is given to pharmaceutically usable ester derivatives which are converted by solvolysis in physiological medium into the original carboxylic acid, such as, for example, lower alkyl esters, cycloalkyl esters, lower alkenyl esters, benzyl esters, mono- or disubstituted lower alkyl esters, such as lower ω-(amino, mono- or dialkylamino, carboxy, lower alkoxycarbonyl)-alkyl esters or such as lower α-(alkanoyloxy, alkoxycarbonyl or dialkylaminocarbonyl)-alkyl esters; pivaloyloxymethyl esters and similar esters are conventionally used as such.

Because of the close relationship between a free compound, a prodrug derivative and a salt compound, a defined compound in this invention also includes its prodrug derivative and salt form where this is possible and appropriate.

Aldosterone is a steroidal hormone which is synthesized in the zona glomerulosa cells of the adrenal cortex by the enzyme aldosterone synthase (CYP11B2). Aldosterone production and secretion is controlled by the adrenocorticotropic hormone (ACTH), angiotensin II, potassium and sodium ions. The primary biological function of aldosterone is to regulate the salt balance, since aldosterone controls the reabsorption of sodium ions from the renal filtrate and the secretion of potassium ions into the renal filtrate. The state of excessive aldosterone secretion, also called hyperaldosteronism, may lead to high blood pressure, hypokalaemia, alkalosis, muscle weakness, polyuria, polydipsia, oedemas, vasculitis, increased collagen formation, fibrosis and endothelial dysfunction.

The chemical compounds described in this invention inhibit the cytochrome P450 enzyme aldosterone synthase (CYP11B2) and can therefore be used to treat states induced by aldosterone. The described compounds can be employed for the prevention, for delaying the progression, or for the treatment of states such as hypokalaemia, hypertension, congestive heart failure, acute and, in particular, chronic renal failure, cardiovascular restenosis, atherosclerosis, metabolic syndrome (syndrome X), adiposity (obesity), vasculitis, primary and secondary hyperaldosteronism, proteinuria, nephropathy, diabetic complications such as diabetic nephropathy, myocardial infarction, coronary heart disease, increased collagen formation, fibrosis, vascular and coronary tissue changes (remodelling) secondary to hypertension, endothelial dysfunction and oedemas secondary to cirrhosis, nephrosis and congestive heart failure.

Cortisol is a steroidal hormone which is synthesized almost exclusively in the zona fasciculata cells of the adrenal cortex by the cytochrome P450 enzyme 11-β-hydroxylase (CYP11B1). Cortisol production is controlled by ACTH. The primary biological function of cortisol is to regulate the production and the availability of carbohydrates for the brain and other metabolically active tissues. Increased cortisol production and secretion is a normal physiological response to stress and leads to the essential mobilization of fats, proteins and carbohydrates to meet an increased demand for energy by the body. Chronically excessive cortisol release describes the condition of Cushing's syndrome. Cushing's syndrome may be produced on the one hand by hypersynthesis of cortisol, which may be generated by an adrenocortical tumour, or be produced on the other hand as the consequence of excessive stimulation of the adrenal cortex by ACTH. The first form is referred to as primary hypercortisolism, and the second form as secondary hypercortisolism. An excessive and persistent cortisol, secretion may also accompany a stress response, which may lead to depression, hyperglycemia and to suppression of the immune system.

The chemical compounds described in this invention inhibit the enzyme 11-β-hydroxylase (CYP11B1) and can therefore, due to the inhibition of cortisol synthesis, be employed for the prevention, delaying the progression or treatment of Cushing's syndrome and of the physical and mental consequences of excessive and persistent cortisol secretion in states of stress. Therefore, these compounds may be useful for the treatment and prevention of conditions such as the ectopic adrenocorticotropic (ACTH) hormone syndrome, adrenal incidentaloma, primary pigmented nodular adrenocortical disease (PPNAD) and Carney complex (CNC), anorexia nervosa, chronic alcohol abuse, cigarette smoking, nicotine and cocaine withdrawal, post-traumatic stress disorder, cognitive dysfunction after stroke and cortisol-mediated mineralcorticoid excess.

Inhibition of aldosterone synthase (Cyp11B2) and of 11-β-hydroxylase (Cyp11B1) and of aromatase (Cyp19), by the compounds described above can be determined by the following *in vitro* assay:

> The cell line NCI-H295R was originally isolated from an adrenocortical carcinoma and has been characterized in the literature through the stimulative secretion of steroid hormones and the presence of the key enzymes necessary for steroidogenesis. These include Cyp11A (cholesterol side-chain cleavage), Cyp11B1 (steroid 11β-hydroxylase), Cyp11B2 (aldosterone synthetase), Cyp17 (steroid 17α-hydroxylase and/or 17,20 lyase), Cyp19 (aromatase), Cyp21 B2 (steroid 21-hydroxylase) and 3β-HSD (hydroxysteroid dehydrogenase). The cells have the physiological characteristics of zonally undifferentiated human fetal adrenal cells, with the ability to produce the steroid hormones of each of the three phenotypically distinct zones found in the adult adrenal cortex.

The NCI-295R cells (American Type Culture Collection, ATCC, Rockville, MD, USA) are cultured in Dulbecco's Modified Eagle'Ham F-12 medium (DME/F12) that is supplemented with Ultroser SF serum (Soprachem, Cergy-Saint-Christophe, France) as well as insulin, transferrin, selenit (I-T-S, Becton Dickinson Biosiences, Franklin Lakes, NJ, USA) and antibiotics in 75 $cm^2$ cell culture flasks at a temperature of 37°C and a 95% air/5% CO2 humidified atmosphere. The cells are subsequently transferred in a 24-well plate and seeded in presence of DME/F12 medium that is supplemented with 0.1% bovine serum albumin instead of Ultroser SF serum. The experiment is initiated by incubating the cells for 72 hours in DME/F12 medium supplemented with 0.1% bovine serum albumin and test compounds in the presence or absence of cell stimulatory agents. The test compound is added in a concentration range of 0.2 nanomolar to 20 millimolar. Angiotensin-II (at 10 or 100 nanomolar concentration), potassium ions (at 16 millimolar), forskolin (at 10 micromolar) or a combination of two agents may serve as cell-stimulatory agents. The cellular secretion of aldosterone, cortisol, corticosterone and estradiol/estrone into the cell culture medium can be quantitatively assessed with commercially available immuno-assays and specific monoclonal antibodies according to the manufacturer's instructions.

The degree of secretion of a selective steroid is used as a measure of enzyme activity, respectively enzyme inhibition in the presence of absence of a test compound. The dose-dependent enzyme inhibitory activity of a compound is reflected in a inhibition curve that is characterized by an IC50 value. The IC50 values for active test compounds are generated by simple linear regression analysis to establish inhibition curves without data weighing. The inhibition curve is generated by fitting a 4-parameter logistic function to the raw data of the samples using the least squares approach. The function is described as follows:

$$Y = (d-a) / ((1 + (x/c)^{-b})) + a$$

with:

- a = minimum
- b = slope
- c= IC50
- d = maximum
- x = inhibitor concentrations

The compounds of the present invention show inhibitory effects in in vitro systems with minimal concentrations of about $10^{-3}$ to about $10^{-10}$ mol/l.

The aldosterone-reducing effect of the compounds described herein can be tested in vivo by the following protocol:

> Adult male Sprague Dawley rats, weighing between 125 and 150 grams, are kept, housed singly, under the usual conditions of light and temperature. At 16.00 h on the first day of the experiment, the animals receive a subcutaneous injection of the depot ACTH product in a dose of 1.0 mg/kg of weight (SYNACTEN-Depot, Novartis, Basel, CH). Pilot studies showed that this ACTH dose increased plasma aldosterone and corticosterone significantly by 15-fold and 25-fold respectively over a period of at least 18 hours. At 8.00 h in the morning of the second day, the animals, divided into test groups of 5 animals, receive administration either of water orally or of a compound in a variable dose range of 0.01-10 mg/kg orally by gavage. Two hours later, blood is taken in EDTA-treated Eppendorf vessels. Plasma samples are obtained by centrifugation of the blood and can be stored at -20°C. An alternative method to stimulate the aldosterone secretion consists in subjecting adult male catherized Wistar rats of 250 to 350 grams weight for 48 hours to a low salt diet and 16 hours prior the start of the experiment with an subcutaneous or intraperitoneal application of furosemide at 10 mg/kg. The furosemide application may be repeated 2 hours prior to the start of the experiment. Pilot studies indicated that this treatment results in a 5 to 20 fold increase in plasma aldosterone levels over a period of 12 to 24 hours. The catheters are chronically implanted in the carotid of the animals and allow thus the periodical sampling of up to 0.2 ml of blood using an AccuSampler (DiLab Europe, Lund, Sweden). The experiment starts with the oral administration of test compound in a dose range of 0.01 to 10 mg/kg. The blood sampling with the AccuSampler occurs 1 hour before the administration of test compound and 2, 4, 6, 8, 12, 16 and 24 hours thereafter. The blood samples are anticoagulated with heparin and centrifuged.

The plasma samples derived form both protocols are tested for the steroid content in previously described radioimmunoassays. The reduction in the steroid levels, such as, for example, aldosterone, serves as a measure of the in vivo bioavailability and enzyme inhibiting activity of the compounds described herein.

The reduction of cardiac damage upon inhibition of the aldosterone synthase with the herein described compounds may be evaluated with the following protocol. The protocol corresponds largely to the protocol described in the publication by Rocha et al. (Endocrinology, Vol. 141, pp 3871-3878, 2000). Adult male Wistar rats are housed in individual

cages and given 0.9% saline as drinking fluid ad libitum throughout the experiment. Three days later, rats are placed on one of the three dosing protocols. Group I (control group with 8 animals) receives for 14 days the nitric oxide synthase inhibiting agent L-NAME (N-nitro-L-arginine methylester, SIGMA, St. Louis, MO, USA). On day 11 of L-NAME treatment, an osmotic minipump containing only saline is implanted in each animal subcutaneously. Group II (L-NAME/Ang II with 8 animals) receives L_NAME for 14 days, and on day 11 of L-NAME treatment, an osmotic minipump containing Ang II is implanted in each animal subcutaneaously. Group III (L-NAME/Ang II/test compound with 8 animals) is treated similarly to group II but receives test compound in a daily dose range of 0.2 to 10 mg/kg rat weight. The test compound is dissolved in distilled water and given by oral gavage; whereas groups I and II receive the vehicle without test compound. The experiment is concluded on day 14 of L-NAME treatment. L-NAME is administered in 0.9% saline containing drinking water at a concentration of 60 mg/100 ml which results in a daily intake of approximately 60 mg/kg. Angiotensin II is administered via Alzet osmotic mini pumps (model 2001, Alza Corp, Palo Alto, CA, USA). The mini-pimp is implanted subcutaneously at the nape of the neck. Angiotensin II (human, 99% peptide purity) is purchased from Sigma Chemical Corp., St. Louis, MO, USA and administered at a dose of 225 ug/kg/day in saline. The concentration of angiotensin II used to fill the pumps is calculated based upon: a) the mean pump rate provided by the manufacturer; b) the body weight of the animals on the day before implantation of the pumps and c) the planned dose. The rats are sacrificed on day 14. Their hearts are removed and sliced through the ventricle/atrium in a "bread-loaf" manner, yielding three samples from the following gross cardiac regions: superior, middle and inferior. The samples are fixed in 10% buffered formalin. Paraffin sections are cut and stained with hematoxyliin/eosin. A single investigator who is blinded to the experimental groups views slides. One slide from each of the three gross cardiac sample regions is analyzed per rat. Cardiac sites (left and right ventricles and the septum) are evaluated separately. The entire section is assessed histologically for the presence of myocardial damage (regardless of the severity) as evidenced by the presence of myocyte necrosis, inflammatory cells, hemorrhages and general tissue disruption. Evaluation of the histological data Is made by comparing groups II and III, i.e. Angiotensin II with or without test compound. The evaluation of the samples may occur semi-quantitatively and can be illustrated with a score table.

The lowering of blood pressure and the reduction of cardiac damage and nephropathy upon inhibition of the aldosterone synthase with the herein described compounds may be evaluated with following protocol. The experiments occur in 4 week old male double transgenic rats (dTGR) that overexpress human angiotensinogen as well as human renin and therefore develop hypertension. Age-paired Sprague-Dawley (SD) rats serve as non-hypertensive control animals. The animals are separated in test groups that receive either test compound or vehicle (control group) for 3-4 week. The animals are fed standard chow and get drinking water ad libitum during the whole experiment. The systolic and diastolic blood pressure as well as the heart rate are monitored with implanted telemetric transducers whereby the animals are free and unrestricted to move. The rats are transferred once a week for 24 hours into a metabolic cage in order to measure the 24 hour urinary albumin excretion. The dimensions of the heart (left ventricular mass, end-diastolic diameter and wall thickness, thickness of the septum, shortening fraction) and the diastolic filling are determined by echocardiography at the beginning and the end of the treatment under isofluran anesthesia (M-mode monitoring in the short axis and tissue Doppler representation using a commercial echocardiogram instrument that is equipped with a 15 MHz probe). The animals are sacrificed at the end of the study and the kidneys and heart removed for weighing and immuno-histochemical assessment (fibrosis, macrophage/T-cell infiltration).

In order to achieve the desired effects in a patient to be treated, the compounds of the present invention can be administered orally or enterally, such as, for example, intravenously, intraperitoneally, intramuscularly, rectally, subcutaneously or else by direct injection of the active substance locally in tissues or tumours. The term patient encompasses warm-blooded species and mammals such as, for example, human, primate, bovine, dog, cat, horse, sheep, mouse, rat and pig. The compounds can be administered as pharmaceutical product or be incorporated into an administration device which ensures permanent release of the compound. The amount of substance to be administered can vary over a wide range and represent every effective dose. Depending on the patient to be treated, or the condition to be treated and mode of administration, the dose of the effective substance each day can be between about 0.005 and 50 milligrams per kilogram of body weight, but is preferably between about 0.05 and 5 milligrams per kilogram of body weight each day.

For oral administration, the compounds can be formulated in solid or liquid pharmaceutical forms such as, for example, as capsules, pills, tablets, coated tablets, granules, powders, solutions, suspensions or emulsions. The dose of a solid pharmaceutical form can be one usual hard gelatin capsule which may be filled with active ingredients and excipients such as lubricants and fillers, such as, for example, lactose, sucrose and maize starch. Another form of administration may be represented by tableting of the active substance of the present invention. The tableting can take place with conventional tableting excipients such as, for example, lactose, sucrose, maize starch, combined with binder from gum acacia, maize starch or gelatin, disintegrants such as potato starch or crosslinked polyvinylpyrrolidone (PVPP) and lubricants such as stearic acid or magnesium stearate.

Examples of excipients suitable for soft gelatin capsules are vegetable oils, waxes, fats, semisolid and liquid polyols etc.

Examples of excipients suitable for producing solutions and syrups are water, polyols, sucrose, invert sugar,

glucose etc.

For rectal administration, the compounds can be formulated in solid or liquid pharmaceutical forms such as, for example, suppositories. Examples of excipients suitable for suppositories are natural or hardened oils, waxes, fats, semiliquid or liquid polyols.

For parenteral administration, the compounds can be formulated as injectable dosage of the active ingredient in a liquid or suspension. The preparations usually comprise a physiologically tolerated sterile solvent which may comprise a water-in-oil emulsion, with or without surfactant, and other pharmaceutically acceptable excipients. Oils which can be used for such preparations are paraffins and triglycerides of vegetable, animal or synthetic origin, such as, for example, peanut oil, soya oil and mineral oil. Injectable solutions generally comprise liquid carriers such as, preferably, water, saline, dextrose or related sugar solutions, ethanol and glycols such as propylene glycol or polyethylene glycol.

The substances may be administered as transdermal patch system, as depot injection or implant if the formulation makes sustained delivery of the active ingredient possible. The active substance can be compressed as granules or to narrow cylinders and be administered subcutaneously or intramuscularly as depot injection or implant.

The pharmaceutical products may in addition also comprise preservatives, solubilizers, viscosity-increasing substances, stabilizers, wetting agents, emulsifiers, sweeteners, colorants, aromatizing agents, salts to change the osmotic pressure, buffers, coating agents or antioxidants. They may also comprise other therapeutically valuable substances too.

The compounds of the invention described herein can be used:

- as therapeutic combination in the form of a product or of a kit which is composed of individual components consisting of a compound described herein, in free form or as pharmaceutically usable salt, and at least one pharmaceutical form whose active ingredient has a blood pressure-lowering, an inotropic, an antidiabetic, an obesity-reducing or a lipid-lowering effect, which can be used either simultaneously or sequentially. The product and the kit may comprise instructions for use.
- for combined use, such as, for example, in simultaneous or sequential succession, of a therapeutically effective amount of a compound described herein, in free or in pharmaceutically usable salt form, and of a second active ingredient with blood pressure-lowering, inotropic, antidiabetic, obesity-reducing or lipid-lowering effect.

The compounds described herein and their pharmaceutically usable salts can be used in combination with

(i) one or more blood pressure-lowering active ingredients, as such for example:

- renin inhibitors such as aliskiren;
- angiotensin II receptor blockers such as candesartan, irbesartan, olmesartan, losartan, valsartan, telmisartan;
- ACE inhibitors such as quinapril, ramipril, trandolapril, lisinopril, captopril, enalapril etc.;
- calcium antagonists such as nifedipine, nicardipine, verapamil, isradipine, nimodipine, amlodipine, felodipine, nisoldipine, diltiazem, fendiline, flunarizine, perhexiline, gallopamil;
- diuretics such as hydrochlorthiazide, chlorothiazide, acetazolamide, amiloride, bumetanide, benzthiazide, etacrynic acid, furosemide, indacrinone, metolazone, triamterene, chlortalidone;
- aldosterone receptor blockers such as spironalactone, eplerenone;
- endothelin receptor blockers such as bosentan;
- phosphodiesterase inhibitors such as amrinone, sildenafil;
- direct vasodilators such as dihydralazine, minoxidil, pinacidil, diazoxide, nitroprusside, flosequinan,
- $\alpha$- and $\beta$-receptor blockers such as phentolamine, phenoxybenzamine, prazosin, doxazosin, terazosin, carvedilol, atenolol, metoprolol, nadolol, propranolol, timolol, carteolol;
- neutral endopeptidase (NEP) inhibitors;
- sympatholytics such as methyldopa, donidine, guanabenz, reserpine

(ii) one or more agents having inotropic activity, as such for example:

- cardiac glycosides such as digoxin;
- $\beta$-preceptor stimulators such as dobutamine
- thyroid hormone such as thyroxine

(iii) one or more agents having antidiabetic activity, as such for example:

- insulins such as insulin aspart, insulin human, insulin lispro, insulin glargine and further fast-, medium- and long-acting insulin derivatives and combinations

- insulin sensitizers such as rosiglitazone, pioglitazone;
- sulphoicnylureas such as glimepiride, chlorpropamide, glipizide, glyburide;
- biguanides such as metformin;
- glucosidase inhibitors such as acarbose, miglitol;
- meglitinides such as repaglinide, nateglinide;

(iv) one or more obesity-reducing ingredients, as such for example:

- lipase inhibitors such as orlistate;
- appetite suppressants such as sibutramine, phentermine;

(v) one or more lipid-lowering active ingredients, such as, for example,

- HMG-CoA reductase inhibitors such as lovastatin, fluvastatin, pravastatin, atorvastatin, simvastatin, rosuvastatin;
- fibrate derivatives such as fenofibrate, gemfibrozil;
- bile acid-binding active ingredients such as colestipol, colestyramine, colesevelam
- cholesterol absorption inhibitors such as ezetimibe
- nicotinic acid such as niacin

and other agents which are suitable for the treatment of high blood pressure, heart failure or vascular disorders associated with diabetes and renal disorders, such as acute or chronic renal failure, in humans and animals. Such combinations can be used separately or in products which comprise a plurality of components.

The presently described compounds and the pharmaceutically usable salts thereof may find use as combinations with

(i) a diagnostic test system, that allows the quantitative determination of the plasma renin concentration (PRC)

(ii) a diagnostic test system, that allows the quantitative determination of the plasma aldosterone concentration (PAC)

(iii) a diagnostic test system, that allows the quantitative determination of the plasma renin activity (PRA)

(iv) a diagnostic test system, that allows the quantitative determination of the plasma aldosterone to renin concentration ratio (ARC)

(v) a diagnostic test system, that allows the quantitative determination of the plasma aldosterone to renin activity ratio (ARR)

(vi) a diagnostic test system, that allows the quantitative determination of the plasma cortisol concentration (PCC)

Such combination of a diagnostic test system and a therapy may be used separately or in preparation with individual components.

The following examples illustrate the present invention. All temperatures are stated in degrees Celsius, pressures in mbar. Unless mentioned otherwise, the reactions take place at room temperature. The abbreviation "Rf = xx (A)" means for example that the Rf is found in solvent system A to have the value xx. The ratio amounts of solvents to one another is always stated in proportions by volume. Chemical names of final products and intermediates were generated with the aid of the AutoNom 2000 (Automatic Nomenclature) program.

HPLC gradients on Hypersil BDS C-18 (5 μm); column: 4 × 125 mm

I 95% water*/5% acetonitrile* to 0% water*/100% acetonitrile* in 10 minutes + 2 minutes at 0% water*/100% acetonitrile* (1 ml/min).

* contains 0.1% trifluoroacetic acid

The following abbreviations are used:

Rf ratio of the distance migrated by a substance to the distance of the solvent from the starting point in thin-layer chromatography

(continued)

| | |
|---|---|
| Rt | retention time of a substance in HPLC (in minutes) |
| m.p. | melting point (temperature) |

Example 1:

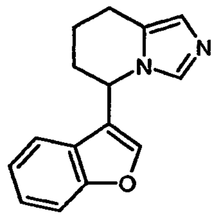

5-Benzofuran-3-yl-5,6,7,8-tetrahydroimidazo[1,5-a]pyridine

1.565 ml of n-butyllithium (1.6M in hexane) are added dropwise to a solution of 2.710 mmol of diisopropylamine in 10 ml of tetrahydrofuran at 0°C, and the mixture is stirred at this temperature for 30 minutes. The solution obtained in this way is slowly added dropwise to a solution of 1.270 mmol of 1-benzofuran-3-ylmethyl-5-(3-chloropropyl)-1H-imidazole and 2.780 mmol of N,N,N',N'-tetramethylenediamine in 10 ml of tetrahydrofuran at -78°C. The reaction mixture is stirred at -78°C for 1 hour and at room temperature for 2 hours and then quenched with saturated aqueous ammonium chloride solution. The organic phase is separated off and the aqueous phase is extracted with dichloromethane (2x). The combined organic phases are dried with sodium sulfate and evaporated. The residue is purified by flash chromatography ($SiO_2$ 60F), and converted into the hydrochloride by adding ethanolic HCl to provide the title compound as an off-white solid. Rf (free base) = 0.45 (dichloromethane-methanol-ammonia conc. 25% = 200:20:1); Rt = 5.68 (I).

The starting materials are prepared as follows:

a) 1-Benzofuran-3-ylmethyl-5-(3-chloropropyl)-1H-imidazole

A solution of 17.947 mmol of 3-(3-benzofuran-3-ylmethyl-3H-imidazol-4-yl)propan-1-ol and 22.434 mmol of thionyl chloride in 100 ml of dichloromethane is heated to reflux for 1 hour. The reaction solution is cooled to room temperature and evaporated. The residue is taken up in dichloromethane and cautiously washed with saturated aqueous sodium bicarbonate solution. The organic phase is separated off, dried over sodium sulfate and evaporated. The crude title compound is obtained as a gold-brown oil from the residue. Rf = 0.40 (toluene/methanol = 85:15); Rt = 6.20 (I).

b) 3-(3-Benzofuran-3-ylmethyl-3H-imidazol-4-yl)propan-1-ol

A solution of 18.240 mmol of 4-(3-trimethylsilanyloxypropyl)imidazole-1-dimethylcarboxamide [102676-27-7] and 18.240 mmol of 3-bromomethylbenzofuran [38281-49-1] in 100 ml of acetonitrile is heated to reflux for 3 hours. The reaction mixture is cooled to room temperature and then, while cooling in ice, gaseous ammonia is passed in for 30 minutes. The reaction mixture is adjusted to pH = 1 by adding aqueous 1 M hydrochloric acid and is washed with diethyl ether (2x). The ether phase is discarded, and the aqueous phase is adjusted to pH = 9 by adding aqueous 2M sodium hydroxide solution and is extracted with dichloromethane (3x). The organic phase is dried over sodium sulfate and evaporated. The crude title compound is obtained as a yellow oil from the residue. Rf = 0.18 (toluene/methanol = 85: 15); Rt = 4.89 (I)

The following compounds are prepared in an analogous manner to the processes described in Example 1:

Examples :

| | |
|---|---|
| 2 | 5-Benzo[b]thiophen-3-yl-5,6,7,8-tetrahydroimidazo[1,5-a]pyridine |

(continued)

| | |
|---|---|
| 3 | 6-(5,6,7,8-Tetrahydroimidazo[1,5-a]pyridin-5-yl)isoquinoline |
| 4 | 5-(6-Chloro-benzo[b]thiophen-3-yl)-5,6,7,8-tetrahydro-imidazo[1,5-a]pyridine |
| 5 | 5-(5-Chloro-benzo[b]thiophen-3-yl)-5,6,7,8-tetrahydro-imidazo[1,5-a]pyridine |
| 6 | 5-(2-Methyl-benzo[b]thiophen-3-yl)-5,6,7,8-tetrahydro-imidazo[1,5-a]pyridine |
| 7 | 5-(1,1-Dioxo-1H-1lambda*6*-benzo[b]thiophen-3-yl)-5,6,7,8-tetrahydro-imidazo[1,5-a]pyridine |
| 8 | 5-(1-Methyl-1H-indol-3-yl)-5,6,7,8-tetrahydro-imidazo[1,5-a]pyridine |
| 9 | 1-Methyl-3-(5,6,7,8-tetrahydro-imidazon[1,5-a]pyridin-5-yl)-1H-indazole |
| 10 | 5-(7-Chloro-benzo[b]thiophen-3-yl)-5,6,7,8-tetrahydro-imidazo[1,5-a]pyridine |
| *11 | 5-(5,6,7,8-Tetrahydro-imidazo[1,5-a]pyidin-5-yl)-thiophene-2-carbonitrile |
| *12 | 5-(5-Phenyl-thiophen-2-yl)-5,6,7,8-tetrahydro-imidazo[1,5-a]pyridine |

* comparative examples

(comparative example) Example 13

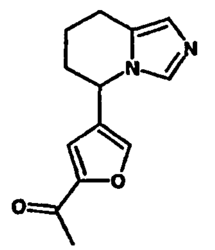

1-[4-(5,6,7,8-Tetrahydroimidazo[1,5-a]pyridin-5-yl)furan-2-yl]ethanone

Starting from 4-(3-trimethylsilanyloxypropyl)imidazcle-1-dimethylcarboxamide [102676-27-7] and 2-(4-bromomethylfuran-2-yl)-2-methyl[1,3]dioxolane.

The starting materials are prepared as follows:

a) 2-(4-Bromomethylfuran-2-yl)-2-methyl[1,3]dioxolane

A stirred solution of 2 mmol of 2-methyl-2-(4-methylfuran-2-yl)-[1,3]dioxolane in 30 ml of tetrachloromethane is heated to reflux and then a mixture of 2.2 mmol of N-bromosuccinimide and 0.2 mmol of benzoyl peroxide is added as solid in portions over a time of 5 minutes. The resulting reaction mixture is heated under reflux for 4.5 hours and cooled to room temperature. Precipitated succinimide is removed by filtering the reaction mixture through Celite, and the solvent is evaporated in vacuo. The title compound is identified from the residue by flash chromatography ($SiO_2$ 60F) on the basis of the Rf.

b) 2-Methyl-2-(4-methylfuran-2-yl)-[1,3]dioxolane

0.2 mmol of p-toluenesulphonic acid is added to a solution of 2 mmol of 1-(4-methylfuran-2-yl)ethanone [33342-43-7] and 2 mmol of methyl orthoformate in 5 ml of ethylene glycol, and the reaction mixture is stirred at 25°C for 16 hours. The solution is then poured into 10 ml of saturated sodium bicarbonate solution and extracted twice with 30 ml of ethyl acetate each time, and the combined organic phases are dried over anhydrous potassium carbonate. The organic phase is filtered and evaporated in vacuo. The title compound is identified from the residue by flash chromatography ($SiO_2$ 60F) on the basis of the Rf.

An alternative possibility for preparing 2-(4-bromomethylfuran-2-yl)-2-methyl[1,3]dioxolane is by the following steps:

c) 2-(4-Bromomethylfuran-2-yl)-2-methyl[1,3]dioxolane

2.2 mmol of bromine are added to a solution of 2.4 mmol of triphenylphosphine in 20 ml of tetrachloromethane at 0°C. The resulting mixture is stirred at 0°C for 10 minutes and then a solution of 2 mmol of [5-(2-methyl[1,3]dioxolan-2-yl)furan-3-yl]methanol in 5 ml of tetrachloromethane is added over the course of 2 minutes. The reaction mixture is stirred at room temperature for 1.5 hours. The solvent is then removed in vacuo. The title compound is identified from the residue by flash chromatography ($SiO_2$ 60F) on the basis of the Rf.

d1) [5-(2-Methyl[1,3]dioxolan-2-yl)furan-3-yl]methanol

3 mmol of a solution of lithium aluminium hydride in tetrahydrofuran (1.0M) are added to a solution of 2 mmol of 5-(2-methyl[1,3]dioxolan-2-yl)furan-3-carboxylic acid [308341-64-2] in 10 ml of tetrahydrofuran at 0°C. The mixture is stirred at room temperature for 30 minutes and then heated at 50°C for 1 hour. Subsequently, at 0°C, water is cautiously added, and the mixture is extracted three times with ethyl acetate. The combined organic phases are dried over sodium sulfate and evaporated. The title compound is identified from the residue by flash chromatography ($SiO_2$ 60F) on the basis of the Rf.

d2) An alternative possibility for preparing [5-(2-methyl[1,3]dioxolan-2-yl)furan-3-yl]methanol is also via the methyl ester of 5-(2-methyl[1,3]dioxolan-2-yl)furan-3-carboxylic acid [308341-64-2] and subsequent reduction with diisobutylaluminium hydride at -78°C in dichloromethane.

The following compounds are prepared in an analogous manner to the processes described in Example 13:

| | |
|---|---|
| *14 | 1-[4-(5,6,7,8-Tetrahydroimidazo[1,5-a]pyridin-5-yl)oxazol-2-yl]ethanone |
| *15 | 1-[3-(5,6,7,8-Tetrahydroimidazo[1,5-a]pyridin-5-yl)pyrrol-1-yl]ethanone |

* Comparative examples

An alternative possibility for preparing 1-[3-(5,6,7,8-tetrahydroimidazo[1,5-a]pyridin-5-yl)pyrrol-1-yl]ethanone is also via the following synthetic route:

1-[3-(5,6,7,8-Tetrahydroimidazo[1,5-a]pyridin-5-yl)pyrrol-1-yl]ethanone

2.2 mmol of acetyl chloride are added to a solution of 2 mmol of 5-(1H-pyrrol-3-yl)-5,6,7,8-tetrahydroimidazo [1,5-a]pyridine, 2.4 mmol of diisopropylethylamine and 0.1 mmol of dimethylaminopyridine in 10 ml of dichloromethane, and the mixture is stirred at room temperature for 18 hours. Then 5 ml of water are added, and the organic phase is washed with saturated sodium bicarbonate solution and brine. The organic phase is dried with sodium sulfate, and the solvent is removed. The title compound is identified from the residue by flash chromatography ($SiO_2$ 60F) on the basis of the Rf.

The starting materials are prepared as follows:

a) 5-(1H-Pyrrol-3-yl)-5,6,7,8-tetrahydromimidazo[1,5-a]pyridine

4 mmol of a solution of tetrabutylammonium fluoride (1.0 M) in tetrahydrofuran are added dropwise to a solution of 2 mmol of 5-(1-triisopropylsilanyl-1H-pyrrol-3-yl)-5,6,7,8-tetrahydroimidazo[1,5-a]pyridine in 10 ml of tetrahydrofuran under argon. After 4 hours 5 ml of water are added to the reaction mixture, and the organic phase is separated off. The organic phase is dried over sodium sulfate and evaporated. The title compound is identified from the residue by flash chromatography ($SiO_2$ 60F) on the basis of the Rf.

b) 5-(1-Triisopropylsilanyl-1H-pyrrol-3-yl)-5,6,7,8-tetrahydroimidazo[1,5-a]pyridine

A solution of 2 mmol of 4-[4-chloro-4-(1-triisopropylsilanyl-1H-pyrrol-3-yl)butyl]-1-trityl-1H-imidazole in 50 ml of acetonitrile is boiled under reflux under argon for 15 hours and then cooled to room temperature. Then 50 ml of methanol are added, and the mixture is boiled under reflux for a further 15 hours. The reaction mixture is evaporated to dryness, and the residue is partitioned between water and ether. The ether phase is separated off and washed twice with 1N aq. HCl. The combined aqueous phases are adjusted to pH 8 and extracted twice with dichloromethane. The combined organic phases are dried over sodium sulfate and evaporated. The title compound is identified from the residue by flash chromatography ($SiO_2$ 60F) on the basis of the Rf.

c1) 4-[4-Chloro-4-(1-triisopropylsilanyl-1H-pyrrol-3-yl)butyl]-1-trityl-1H-imidazole

A solution of 2 mmol of 1-(1-triisopropylsilanyl-1H-pyrrol-3-yl)-4-(1-trityl-1H-imidazol-4-yl)butan-1-ol and 6 mmol of thionyl chloride in 20 ml of dichloromethane is boiled under reflux for 1 hour, cooled and poured into 3 ml of a saturated, ice-cold sodium bicarbonate solution. The organic phase is separated off, dried over sodium sulfate and evaporated. The title compound is identified from the residue by flash chromatography ($SiO^2$ 60F) on the basis of the Rf.

An alternative possibility for preparing 4-[4-chloro-4-(1-triisopropylsilanyl-1H-pyrrol-3-yl)butyl]-1-trityl-1H-imidazole is by the following procedure:

c2) A solution of 2 mmol of 1-(1-triisopropylsilanyl-1H-pyrrol-3-yl)-4-(1-trityl-1H-imidazol-4-yl)butan-1-ol and 2 mmol of triphenylphosphine in 5 ml of tetrachloromethane is heated under reflux for 4 hours. The reaction mixture is cooled to room temperature and then evaporated to dryness. The title compound is identified from the residue by flash chromatography ($SiO_2$ 60F) on the basis of the Rf.

d) 1-(1-Triisopropylsilanyl-1H-pyrrol-3-yl)-4-(1-trityl-1H-imidazol-4-yl)butan-1-ol

2 mmol of a solution of n-butyllithium (1.6M) in hexane are added dropwise to a solution of 2 mmol of 3-bromo-1-triisopropylsilanyl-1H-pyrrole [87630-36-2] in 30 ml of tetrahydrofuran at -23°C under argon. After stirring at -23°C for 2 hours, a solution of 4 mmol of 4-(1-trityl-1H-imidazol-4-yl)butyraldehyde [184030-88-4] in 10 ml of tetrahydrofuran is slowly added at -23°C. The reaction mixture is warmed to room temperature over the course of 20 minutes and quenched with 2 ml of water. The mixture is extracted three times with ethyl acetate, and the combined organic phases are dried over sodium sulfate and evaporated. The title compound is identified from the residue by flash chromatography ($SiO_2$ 60F) on the basis of the Rf.

## Claims

1. A compound of the general formula

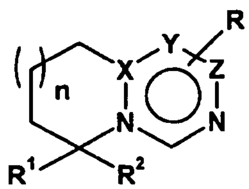

(I),

wherein
X is C;
Y is C;
Z is a bond;
R is hydrogen, $C_1$-$C_8$-alkyl, $C_1$-$C_8$-alkoxy-$C_0$-$C_4$-alkyl, halogen or trifluoromethyl;
$R^1$ is unsaturated heterocyclyl-$C_0$-$C_4$-alkyl, which radical is unsubstituted or substituted by 1-4 $C_1$-$C_8$-alkyl, $C_0$-$C_8$-alkylcarbonyl, $C_1$-$C_8$-alkylsulfonyl, halogen, cyano, oxo, tri-$C_1$-$C_4$-alkylsilyl, trifluoromethoxy, trifluoromethyl, $C_0$-$C_8$-alkylcarbonylamino, $C_0$-$C_8$-alkylcarbonyl-$C_1$-$C_8$-alkylamino, carbamoyl, mono- or di-$C_1$-$C_8$-alkylaminocarbonyl, carboxy-$C_0$-$C_4$-alkyl, $C_1$-$C_8$-alkoxy, $C_1$-$C_8$-alkoxycarbonyl, heterocyclyl or aryl, whereby heterocyclyl or aryl is unsubstituted or substituted by 1-4 $C_1$-$C_8$-alkyl, $C_0$-$C_8$-alkylcarbonyl, $C_1$-$C_8$-alkylsulfonyl, halogen, cyano, oxo, tri-$C_1$-$C_4$-alkylsilyl, trifluoromethoxy, trifluoromethyl, $C_0$-$C_8$-alkylcarbonylamino, $C_0$-$C_8$-alkylcarbonyl-$C_1$-$C_8$-alkylamino, carbamoyl, mono- or di-$C_1$-$C_8$-alkylaminocarbonyl, carboxy-$C_0$-$C_4$-alkyl, $C_1$-$C_8$-alkoxy or $C_1$-$C_8$-alkoxycarbonyl;
$R^2$

a) is hydrogen; or
b) is $C_1$-$C_8$-alkyl, $C_3$-$C_8$-cycloalkyl, halogen, carboxy-$C_1$-$C_4$-alkyl, $C_1$-$C_4$-alkoxycarbonyl-$C_1$-$C_4$-alkyl, $C_0$-$C_4$-alkylcarbonyl, aryl-$C_0$-$C_4$-alkyl or unsaturated heterocyclyl-$C_1$-$C_4$-alkyl, which radicals are unsubstituted or substituted by 1-4 $C_1$-$C_8$-alkyl, $C_0$-$C_8$-alkylcarbonyl, $C_1$-$C_8$-alkylsulfonyl, halogen, cyano, oxo, tri-$C_1$-$C_4$-alkylsilyl, trifluoromethoxy, trifluoromethyl, $C_0$-$C_8$-alkylcarbonylamino, $C_0$-$C_8$-alkylcarbonyl-

$C_1$-$C_8$-alkylamino, carbamoyl, mono- or di-$C_1$-$C_8$-alkylaminocarbonyl, carboxy-$C_0$-$C_4$-alkyl, $C_1$-$C_8$-alkoxy, $C_1$-$C_8$-alkoxycarbonyl, heterocyclyl or aryl, where heterocyclyl or aryl is unsubstituted or substituted by 1-4 $C_1$-$C_8$-alkyl, $C_0$-$C_8$-alkylcarbonyl, $C_1$-$C_8$-alkylsulfonyl, halogen, cyano, oxo, tri-$C_1$-$C_4$-alkylsilyl, trifluoromethoxy, trifluoromethyl, $C_0$-$C_8$-alkylcarbonylamino, $C_0$-$C_8$-alkylcarbonyl-$C_1$-$C_8$-alkylamino, carbamoyl, mono- or di-$C_1$-$C_8$-alkylaminocarbonyl, carboxy-$C_0$-$C_4$-alkyl, $C_1$-$C_8$-alkoxy or $C_1$-$C_8$-alkoxycarbonyl;

n is a number 0, 1 or 2;
and pharmaceutically usable salts thereof,
and its salt or compound in which one or more atoms are replaced by their stable, nonradioactive isotopes; wherein heterocyclyl stands for an unsaturated 7-12 membered bicyclic ring system; whereby, if $R^2$ is hydrogen, $R^1$ is not benzoimidazolyl or benzotriazolyl.

**2.** A compound according to Claim 1, which corresponds to the general formula

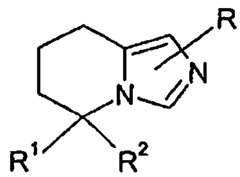

(Ia),

whereby the meanings of the substituents R, $R^1$ and $R^2$ are as indicated for compounds of the formula (I) according to claim 1.

**3.** A compound according to Claim 1 or 2, wherein R is hydrogen, $C_1$-$C_8$-alkyl, halogen or trifluoromethyl.

**4.** A compound according to any of Claims 1 to 3, wherein $R^1$ is indolyl, indazolyl, benzofuranyl, benzothiophenyl or isoquinolinyl, which radicals are unsubstituted or substituted by 1-4 $C_1$-$C_8$-alkyl, $C_0$-$C_8$-alkylcarbonyl, halogen, cyano, oxo, trifluoromethyl, $C_0$-$C_8$-alkylcarbonylamino, $C_0$-$C_8$-alkylcarbonyl-$C_1$-$C_8$-alkylamino, carbamoyl, mono- or di-$C_1$-$C_8$-alkylaminocarbonyl, carboxy-$C_0$-$C_4$-alkyl, $C_1$-$C_8$-alkoxy, $C_1$-$C_8$-alkoxycarbonyl, heterocyclyl or aryl.

**5.** A compound according to any of Claims 1 to 4, wherein $R^2$ is hydrogen, halogen, $C_1$-$C_8$-alkyl, aryl-$C_0$-$C_4$-alkyl or unsaturated heterocyclyl-$C_0$-$C_4$-alkyl.

**6.** A compound according to Claim 1, wherein n is a number 0 or 1.

**7.** A compound according to Claim 1 or 2, wherein
R is hydrogen or methyl;
$R^1$ is indolyl, indazolyl, benzofuranyl, benzothiophenyl or isoquinolinyl, which radicals are unsubstituted or substituted by 1-4 $C_1$-$C_8$-alkyl, $C_0$-$C_8$-alkylcarbonyl, halogen, cyano, oxo, trifluoromethyl, $C_0$-$C_8$-alkylcarbonylamino, $C_0$-$C_8$-alkylcarbonyl-$C_1$-$C_8$-alkylamino, carbamoyl, mono- or di-$C_1$-$C_8$-alkylaminocarbonyl, carboxy-$C_0$-$C_4$-alkyl, $C_1$-$C_8$-alkoxy, $C_1$-$C_8$-alkoxycarbonyl, heterocyclyl or aryl; and
$R^2$ is hydrogen or $C_1$-$C_3$-alkyl.

**8.** A compound of the general formula (I) or (Ia) according to any of Claims 1 to 7 for use as a medicament.

**9.** Use of a compound of the general formula (I) or (Ia) according to any of Claims 1 to 7, for producing a human medicament for the prevention, for delaying the progression or for the treatment of pathological states which are caused or partly caused by hyperaldosteronism.

**10.** Use of a compound of the general formula (I) or (Ia) according to any of Claims 1 to 7, for producing a medicament for the prevention, for delaying the progression or for the treatment of pathological states which are caused or partly caused by excessive cortisol release.

**11.** A compound of the general formula (I) or (Ia) according to any of claims 1 to 7 for the prevention, for delaying the progression or for the treatment of pathological states which are caused or partly caused by hyperaldosteronism.

**12.** A compound of the general formula (I) or (Ia) according to any of claims 1 to 7 for the prevention, for delaying the progression or for the treatment of pathological states which are caused or partly caused by excessive cortisol release.

**13.** A pharmaceutical product comprising a compound of the general formula (I) or (Ia) according to any of Claims 1 to 7, and conventional excipients.

**14.** A pharmaceutical combination in the form of a product or of a kit composed of individual components consisting a) of a compound of the general formula (I) or (Ia) according to any of Claims 1 to 7, and b) at least one pharmaceutical form whose active ingredient has a blood pressure-lowering, an inotropic, a metabolic or a lipid-lowering effect.

**Patentansprüche**

**1.** Verbindung der allgemeinen Formel

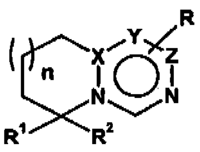

(I),

worin
X für C steht;
Y für C steht;
Z für eine Bindung steht.
R für Wasserstoff, $C_1$-$C_8$-Alkyl, $C_1$-$C_8$-Alkoxy-$C_0$-$C_4$-alkyl, Halogen oder Trifluormethyl steht;
$R^1$ für nicht gesättigtes Heterocyclyl-$C_0$-$C_4$-alkyl steht, wobei der Rest nicht substituiert ist oder durch 1-4 $C_1$-$C_8$-Alkyl, $C_0$-$C_8$-Alkyloarbonyl, $C_1$-$C_8$-Alkylsulfonyl, Halogen, Cyano, Oxo, Tri-$C_1$-$C_4$-Alkylsllyl, Trlfluormethoxy, Trifluormethyl, $C_0$-$C_8$-Alkylcarbonylamino, $C_0$-$C_8$-Alkylcarbonyl-$C_1$-$C_8$-alkylamino, Carbamoyl, Mono- oder Di-$C_1$-$C_8$-alkylamlnocarbonyl, Carboxy-$C_0$-$C_4$-alkyl, $C_1$-$C_8$-Alkoxy, $C_1$-$C_8$-Alkoxycarbonyl, Heterocyclyl oder Aryl substituiert ist, wobei Heterocyclyl oder Aryl nicht substituiert ist oder durch 1-4 $C_1$-$C_8$-Alkyl, $C_0$-$C_8$-Alkylcarbonyl, $C_1$-$C_8$-Alkylsulfonyl, Halogen, Cyano, Oxo, Tri-$C_1$-$C_4$-Alkylsilyl, Trifluormethoxy, Trifluormethyl, $C_0$-$C_8$-Alkylcarbonylamlno, $C_0$-$C_8$-Alkylcarbonyl-$C_1$-$C_8$-Alkylamino, Carbamoyl, Mono- oder Di-$C_1$-$C_8$-Alkylaminocarbonyl, Carboxy-$C_0$-$C_4$-alkyl, $C_1$-$C_8$-Alkoxy oder $C_1$-$C_8$-Alkoxycarbonyl substituiert ist;
$R^2$

a) für Wasserstoff steht; oder
b) für $C_1$-$C_8$-Alkyl, $C_3$-$C_8$-Cycloalkyl, Halogen, Carboxy-$C_1$-$C_4$-alkyl, $C_1$-$C_4$-Alkoxycarbonyl-$C_1$-$C_4$-alkyl, $C_0$-$C_4$-Alkylcarbonyl, Aryl-$C_0$-$C_4$-Alkyl oder nicht gesättigtes Heterocyclyl-$C_1$-$C_4$-alkyl steht, wobei die Reste nicht substituiert sind oder durch 1-4 $C_1$-$C_8$-Alkyl, $C_0$-$C_8$-Alkylcarbonyl, $C_1$-$C_8$-Alkylsulfonyl, Halogen, Cyano, Oxo, Tri-$C_1$-$C_4$-alkylsilyl, Trifluormethoxy, Trifluormethyl, $C_0$-$C_8$-Alkylcarbonylamino, $C_0$-$C_8$-Alkylcarbonyl-$C_1$-$C_8$-alkylamino, Carbamoyl, Mono- oder Di-$C_1$-$C_8$-alkylaminocarbonyl, Carboxy-$C_0$-$C_4$-alkyl, $C_1$-$C_8$-Alkoxy, $C_1$-$C_8$-Alkoxycarbonyl, Heterocyclyl oder Aryl substituiert sind, wobei Heterocyclyl oder Aryl nicht substituiert ist oder durch 1-4 $C_1$-$C_8$-Alkyl, $C_0$-$C_8$-Alkylcarbonyl, $C_1$-$C_8$-Alkylsulfonyl, Halogen, Cyano, Oxo, Tri-$C_1$-$C_4$-alkylsilyl, Trifluormethoxy, Trifluormethyl, $C_0$-$C_8$-Alkylcarbonylamino, $C_0$-$C_8$-Alkylcarbonyl-$C_1$-$C_8$-alkylamino, Carbamoyl, Mono- oder Di-$C_1$-$C_8$-alkylaminocarbonyl, Carboxy-$C_0$-$C_4$-alkyl, $C_1$-$C_8$-Alkoxy oder $C_1$-$C_8$-Alkoxycarbonyl substituiert ist;

n für eine Zahl von 0, 1 oder 2 steht;
und pharmazeutisch geeignete Salze hiervon und ein Salz oder eine Verbindung hiervon, worin ein oder mehrere Atome durch ihre stabilen, nicht radioaktiven Isotope ersetzt ist bzw. sind; wobei Heterocyclyl für ein nicht gesättigtes 7- bis 12-gliedrlges bizyklisches Ringsystem steht, wobei, wenn $R^2$ für Wasserstoff steht, $R^1$ nicht Benzoimidazolyl oder Benzotriazolyl bedeutet.

**2.** Verbindung nach Anspruch 1, die der folgenden allgemeinen Formel entspricht:

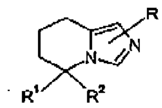
(Ia),

wobei die Bedeutungen der Substituenten R, $R^1$ und $R^2$ die für Verbindungen der Formel (I) gemäß Anspruch 1 angegebenen sind.

3. Verbindung nach Anspruch 1 oder 2, worin R für Wasserstoff, $C_1$-$C_8$-Alkyl, Halogen oder Trifluormethyl steht.

4. Verbindung nach einem der Ansprüche 1 bis 3, worin $R^1$ für Indolyl, Indazolyl, Benzofuranyl, Benzothiophenyl oder Isochinolinyl steht, wobei die Reste nicht substituiert sind oder durch 1-4 $C_1$-$C_8$-Alkyl, $C_0$-$C_8$-Alkylcarbonyl, Halogen, Cyano, Oxo, Trifluormethyl, $C_0$-$C_8$-Alkylcarbonylamino, $C_0$-$C_8$-Alkylcarbonyl-$C_1$-$C_8$-alkylamino, Carbamoyl, Mono- oder Di-$C_1$-$C_8$-alkylaminocarbonyl, Carboxy-$C_0$-$C_4$-alkyl, $C_1$-$C_8$-Alkoxy, $C_1$-$C_8$-Alkoxycarbonyl, Heterocyclyl oder Aryl substituiert sind.

5. Verbindung nach einem der Ansprüche 1 bis 4, worin $R^2$ für Wasserstoff, Halogen, $C_1$-$C_6$-Alkyl, Aryl-$C_0$-$C_4$-alkyl oder nicht gesättigtes Heterocyclyl-$C_0$-$C_4$-alkyl steht.

6. Verbindung nach Anspruch 1, worin n für eine Zahl von 0 oder 1 steht.

7. Verbindung nach Anspruch 1 oder 2, worin
R für Wasserstoff oder Methyl steht;
$R^1$ für Indolyl, Indazolyl, Benzofuranyl, Benzothiophenyl oder Isochinolinyl steht, wobei die Reste nicht substituiert sind oder durch 1-4 $C_1$-$C_8$-Alkyl, $C_0$-$C_8$-Alkylcarbonyl, Halogen, Cyano, Oxo, Trifluormethyl, $C_0$-$C_8$-Alkylcarbonylamino, $C_0$-$C_8$-Alkylcarbonyl-$C_1$-$C_8$-alkylamino, Carbamoyl, Mono- oder Di-$C_1$-$C_8$-alkylaminocarbonyl, Carboxy-$C_0$-$C_4$-alkyl, $C_1$-$C_8$-Alkoxy, $C_1$-$C_8$-Alkoxycarbonyl, Heterocyclyl oder Aryl substituiert sind; und
$R^2$ für Wasserstoff oder $C_1$-$C_3$-Alkyl steht.

8. Verbindung der allgemeinen Formel (I) oder (Ia) nach einem der Ansprüche 1 bis 7 zur Verwendung als Medikament.

9. Verwendung einer Verbindung der allgemeinen Formel (I) oder (Ia) nach einem der Ansprüche 1 bis 7 zur Herstellung eines Humanmedikaments zur Verhinderung, zur Verzögerung des Fortschreitens oder zur Behandlung von pathologischen Zuständen, die durch Hyperaldosteronismus verursacht oder teilweise verursacht sind.

10. Verwendung einer Verbindung der allgemeinen Formel (I) oder (Ia) nach einem der Ansprüche 1 bis 7 zur Herstellung eines Medikaments zur Verhinderung, zur Verzögerung des Fortschreitens oder zur Behandlung von pathologischen Zuständen, die durch übermäßige Cortlsolfrelsetzung verursacht oder teilweise verursacht sind.

11. Verbindung der allgemeinen Formel (I) oder (Ia) nach einem der Ansprüche 1 bis 7 zur Verhinderung, zur Verzögerung des Fortschreitens oder zur Behandlung von pathologischen Zuständen, die durch Hyperaldosteronismus verursacht oder teilweise verursacht sind.

12. Verbindung der allgemeinen Formel (I) oder (Ia) nach einem der Ansprüche 1 bis 7 zur Verhinderung, zur Verzögerung des Fortschreitens oder zur Behandlung von pathologischen Zuständen, die durch übermäßige Cortisolfreisetzung verursacht oder teilweise verursacht sind.

13. Pharmazeutisches Produkt, das eine Verbindung der allgemeinen Formel (I) oder (Ia) nach einem der Ansprüche 1 bis 7 und herkömmliche Hilfsstoffe umfasst.

14. Pharmazeutische Kombination in Form eines Produkts oder eines Kits aus individuellen Komponenten, die aus a) einer Verbindung der allgemeinen Formel (I) oder (Ia) nach einem der Ansprüche 1 bis 7 und b) mindestens einer pharmazeutischen Form bestehen, deren wirksamer Bestandteil eine blutdrucksenkende, inotrope, metabolische oder lipidsenkende Wirkung aufweist.

## Revendications

**1.** Composé de formule générale

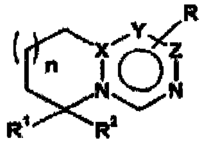

(I),

dans laquelle
X représente C;
Y représente C;
Z est une liaison;
R est un atome d'hydrogène, un radical alkyle en $C_1$-$C_8$, alcoxy(en $C_1$-$C_8$)-alkyle en $C_0$-$C_4$, halogène ou trifluorométhyle;
$R^1$ est un radical hétérocyclyl(insaturé)-alkyle en $C_0$-$C_4$, lequel radical est non substitué ou substitué par 1-4 substituants alkyle en $C_1$-$C_8$, alkyl(en $C_0$-$C_8$)carbonyle, alkyl(en $C_1$-$C_8$)sulfonyle, halogène, cyano, oxo, tri-alkyl(en $C_1$-$C_4$) silyle, trifluorométhoxy, trifluorométhyle, alkyl(en $C_0$-$C_8$)carbonylamino, alkyl(en $C_0$-$C_8$)carbonyl-alkyl(en $C_1$-$C_8$) amino, carbamoyle, mono- ou di-alkyl(en $C_1$-$C_8$)aminocarbonyle, carboxy-alkyle en $C_0$-$C_4$, alcoxy en $C_1$-$C_8$, alcoxy (en $C_1$-$C_8$)carbonyle, hétérocyclyle ou aryle, où le groupe hétérocyclyle ou aryle est non substitué ou substitué par 1-4 substituants alkyle en $C_1$-$C_8$, alkyl(en $C_0$-$C_8$)carbonyle, alkyl(en $C_1$-$C_8$)sulfonyle, halogène, cyano, oxo, tri-alkyl (en $C_1$-$C_4$)silyle, trifluorométhoxy, trifluorométhyle, alkyl(en $C_0$-$C_8$)carbonylamino, alkyl(en $C_0$-$C_8$)carbonyl-alkyl(en $C_1$-$C_8$)amino, carbamoyle, mono- ou di-alkyl(en $C_1$-$C_8$)aminocarbonyle, carboxy-alkyle en $C_0$-$C_4$, alcoxy en $C_1$-$C_8$ ou alcoxy(en $C_1$-$C_8$)carbonyle;
$R^2$ est

a) un atome d'hydrogène; ou
b) un radical alkyle en $C_1$-$C_8$, cycloalkyle en $C_3$-$C_8$, halogène, carboxy-alkyle en $C_1$-$C_4$, alcoxy(en $C_1$-$C_4$) carbonyl-alkyle en $C_1$-$C_4$, alkyl(en $C_0$-$C_4$)carbonyle, aryl-alkyle en $C_0$-$C_4$ ou hétérocyclyl(insaturé)-alkyle en $C_1$-$C_4$, lesquels radicaux sont non substitués ou substitués par 1-4 substituants alkyle en $C_1$-$C_8$, alkyl(en $C_0$-$C_8$) carbonyle, alkyl(en $C_1$-$C_8$)sulfonyle, halogène, cyano, oxo, tri-alkyl(en $C_1$-$C_4$)silyle, trifluorométhoxy, trifluorométhyle, alkyl(en $C_0$-$C_8$)carbonylamino, alkyl(en $C_0$-$C_8$)carbonyl-alkyl(en $C_1$-$C_8$)amino, carbamoyle, mono- ou di-alkyl(en $C_1$-$C_8$)aminocarbonyle, carboxy-alkyle en $C_0$-$C_4$, alcoxy en $C_1$-$C_8$, alcoxy(en $C_1$-$C_8$)carbonyle, hétérocyclyle ou aryle, où le groupe hétérocyclyle ou aryle est non substitué ou substitué par 1-4 substituants alkyle en $C_1$-$C_8$, alkyl(en $C_0$-$C_8$)carbonyle, alkyl(en $C_1$-$C_8$)sulfonyle, halogène, cyano, oxo, tri-alkyl(en $C_1$-$C_4$) silyle, trifluorométhoxy, trifluormethyle, alkyl(en $C_0$-$C_8$)carbonylamino, alkyl(en $C_0$-$C_8$)carbonyl-alkyl(en $C_1$-$C_8$) amino, carbamoyle, mono- ou di-alkyl(en $C_1$-$C_8$)aminocarbonyle, carboxy-alkyle en $C_0$-$C_4$, alcoxy en $C_1$-$C_8$ ou alcoxy(en $C_1$-$C_8$)carbonyle;

n est un nombre égal à 0, 1 ou 2;
et les sels pharmaceutiquement utilisables de celui-ci,
et son sel ou composé dans lequel un ou plusieurs atomes sont remplacés par leurs isotopes stables, non radioactifs;
ou hétérocyclyle signifie un système à noyau bicyclique insaturé ayant 7-12 chaînons;
où, si $R^2$ est un atome d'hydrogène, $R^1$ n'est ni un benzoimidazolyle ni un benzotriazolyle.

**2.** Composé selon la revendication 1, qui correspond à la formule générale

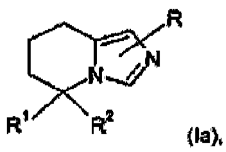

(Ia),

où les significations des substituants R, $R^1$ et $R^2$ sont telles qu'indiquées pour les composés de formule (I) selon

la revendication 1.

3. Composé selon la revendication 1 ou 2, dans lequel R est un atome d'hydrogène, un radical alkyle en $C_1$-$C_8$, halogène ou trifluorométhyle.

4. Composé selon l'une quelconque des revendications 1 à 3, dans lequel $R^1$ est un radical indolyle, indazolyle, benzofuranyle, benzothiophényle ou isoquinoléinyle, lesquels radicaux sont non substitués ou substitués par 1-4 substituants alkyle en $C_1$-$C_8$, alkyl(en $C_0$-$C_8$)carbonyle, halogène, cyano, oxo, trifluorométhyle, alkyl(en $C_0$-$C_8$) carbonylamino, alkyl(en $C_0$-$C_8$)carbonyl-alkyl(en $C_1$-$C_8$)amino, carbamoyle, mono- ou di-alkyl(en $C_1$-$C_8$)aminocarbonyle, carboxy-alkyle en $C_0$-$C_4$, alcoxy en $C_1$-$C_8$, alcoxy(en $C_1$-$C_8$)carbonyle, hétérocyclyle ou aryle.

5. Composé selon l'une quelconque des revendications 1 à 4, dans lequel $R^2$ est un atome d'hydrogène, un atome d'halogène, un radical alkyle en $C_1$-$C_8$, aryl-alkyle en $C_0$-$C_4$ ou hétérocyclyl(insaturé)-alkyle en $C_0$-$C_4$.

6. Composé selon la revendication 1, dans lequel n est un nombre égal à 0 ou 1.

7. Composé selon la revendication 1 ou 2, dans lequel
R est un atome d'hydrogène ou un groupe méthyle;
$R^1$ est un radical indolyle, indazolyle, benzofuranyle, benzothiophényle ou isoquinoléinyle, lesquels radicaux sont non substitués ou substitués par 1-4 substituants alkyle en $C_1$-$C_8$, alkyl(en $C_0$-$C_8$)carbonyle, halogène, cyano, oxo, trifluorométhyle, alkyl(en $C_0$-$C_8$)carbonylamino, alkyl(en $C_0$-$C_8$)carbonyl-alkyl(en $C_1$-$C_8$)amino, carbamoyle, mono- ou di-alkyl(en $C_1$-$C_8$)aminocarbonyle, carboxy-alkyle en $C_0$-$C_4$, alcoxy en $C_1$-$C_8$, alcoxy(en $C_1$-$C_8$)carbonyle, hétérocyclyle ou aryle; et
$R^2$ est un atome d'hydrogène ou un radical alkyle en $C_1$-$C_3$.

8. Composé de formule générale (I) ou (Ia) selon l'une quelconque des revendications 1 à 7, à utiliser comme médicament.

9. Utilisation d'un composé de formule générale (I) ou (Ia) selon l'une quelconque des revendications 1 à 7, pour la production d'un médicament destiné à l'humain pour la prévention, pour retarder la progression ou pour le traitement d'états pathologiques qui sont dus ou partiellement dus à un hyperaldostéronisme.

10. Utilisation d'un composé de formule générale (I) ou (Ia) selon l'une quelconque des revendications 1 à 7, pour la production d'un médicament pour la prévention, pour retarder la progression ou pour le traitement d'états pathologiques qui sont dus ou partiellement dus à une libération excessive de cortisol.

11. Composé de formule générale (I) ou (Ia) selon l'une quelconque des revendications 1 à 7 pour la prévention, pour retarder la progression ou pour le traitement d'états pathologiques qui sont dus ou partiellement dus à un hyperaldostéronisme.

12. Composé de formule générale (I) ou (Ia) selon l'une quelconque des revendications 1 à 7 pour la prévention, pour retarder la progression ou pour le traitement d'états pathologiques qui sont dus ou partiellement dus à une libération excessive de cortisol.

13. Produit pharmaceutique comprenant un composé de formule générale (I) ou (Ia) selon l'une quelconque des revendications 1 à 7, et des excipients classiques.

14. Combinaison pharmaceutique sous la forme d'un produit ou d'une trousse composée de composants individuels constitués a) d'un composé de formule générale (I) ou (Ia) selon l'une quelconque des revendications 1 à 7, et b) d'au moins une forme pharmaceutique dont le principe actif a un effet hypotenseur, inotrope, métabolique ou hypolipémiant.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 09071586 A
- EP 0366609 A


### Non-patent literature cited in the description


- **Rocha et al.** *Endocrinology,* 2000, vol. 141, 3871-3878